# DEMANDE DE BREVET EUROPEEN

(11) **EP 2 374 470 A1**
(43) Date de publication de la demande: **12.10.2011**
(21) Numéro de dépôt: 10290188.1
(22) Date de dépôt: 08.04.2010
(51) Int. Cl.: A61K 38/17, C12Q 1/00, A61P 37/06

(54) **Utilisation thérapeutique de la protéine Beta2m**

(71) Demandeur: Beta Innov, 34960 Montpellier (FR)
(72) Inventeur: Mersel, Marcel, 34090 Montpellier (FR); Rakotoarivelo, Clovis, 34070 Montpellier (FR)
(74) Mandataire: Santarelli

(57) **Abrégé**

La présente invention concerne l'utilisation de la beta2-microglobuline (β2m) en tant que principe actif, notamment dans des compositions pharmaceutiques destinées au traitement des maladies auto-immunes.

## Description

La présente demande de brevet concerne le domaine médical, notamment celui du traitement des maladies auto-immunes.

L'invention traite plus particulièrement de l'utilisation de la beta2-microglobuline (β2m) en tant que principe actif, notamment dans des compositions pharmaceutiques destinées au traitement des maladies auto-immunes telle que, par exemple, la sclérose en plaques ou la maladie de Crohn.

### PREAMBULE

La protéine β2m est une protéine ayant un poids moléculaire moyen d'environ 11,6 kDa, formée d'environ 99 acides aminés, qui entre dans la constitution des complexes majeurs d'histocompatibilité (MHC I ou HLA I) « Human Leucocytes Antigens) [Cunningham B.A. et a/. The complete amino acid sequence of beta-2-microglobulin (1973) Biochemistry 12: 4811-4821].

Il est rappelé que les complexes d'histocompatibilité MHC I jouent un rôle central dans la reconnaissance du «soi» et du «non-soi» par le système immunitaire. Ces complexes sont présents à la surface de la plupart des cellules humaines, à l'exception des hématies. Ils présentent à leur surface un nombre important d'antigènes à partir desquels les lymphocytes T (CD8) sont capables de discriminer des cellules d'un individu, des cellules étrangères à celui-ci, malades ou subissant un processus de transformation tumoral.

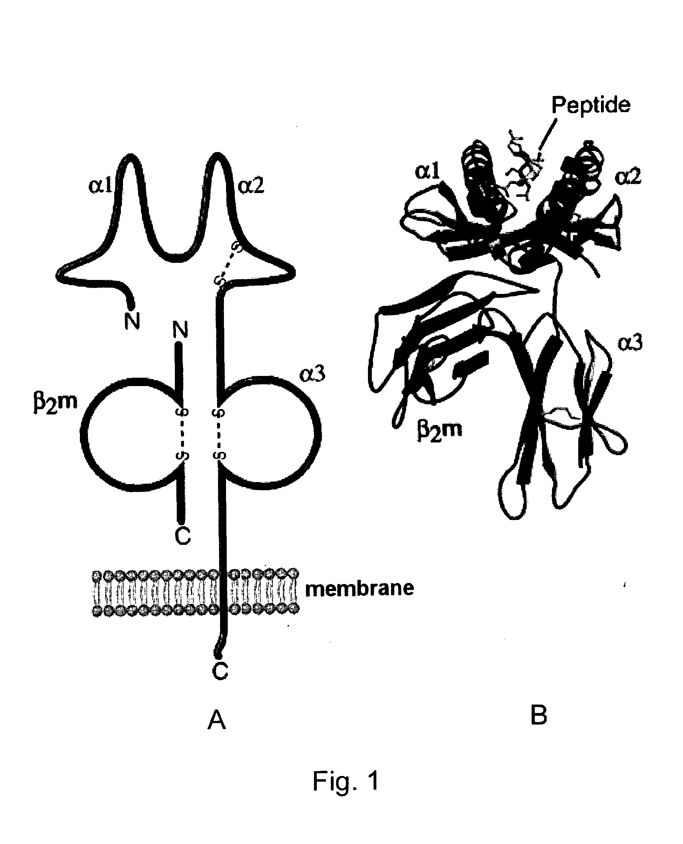

Les complexes MHC I sont composés d'une chaine lourde (CL) glycosylée, d'environ 44kDa, et d'une chaîne légère, la β2m, qui s'associe au domaine extracellulaire de la chaîne lourde de manière non covalente. La chaine α de MHC I (CL) est composée de trois domaines extracellulaires (α1, α2 et α3) et d'un segment transmembranaire comme indiqué dans la Figure 1A. La β2m s'associe à une séquence d'acides aminés qui se situe dans la zone de rapprochement entre l'extrémité du domaine α1 et le début du domaine α3 de CL [Gussov, D. et a/. The human beta-2-microglobulin gene: primary structure and definition of transcriptional unit (1987) Journal of Immunology 139:3132-3138]. Selon l'ordre de leur découverte, les gènes codant les molécules MHC I ont été numérotés et classés par groupes (A, B et C) et complexes (D, H et G).

Les cellules présentatrices d'antigène (CPA) utilisent les complexes de type MHC I en tant que présentoirs d'antigènes pour les cellules T (CD8) du système immunitaire. Les antigènes présentés par MHC I sont généralement constitués d'une variété de polypeptides ayant 8 à 10 acides aminés, qui résultent du fractionnement de protéines endogènes par le protéasome. Ces antigènes sont chargés sur les sous-unités (CL et β2m) au cours de la formation des complexes MHC I à l'intérieur du réticulum endoplasmique. Une fois les antigènes chargés, les complexes MHC sont ensuite exportés à la surface de la cellule. L'ancrage du complexe MHC I dans la membrane plasmique est alors assuré par le domaine transmembranaire de la chaîne lourde situé au niveau du domaine α3.

La sous-unité formée par la protéine β2m se distingue des chaînes lourdes en ce qu'elle est invariable et n'est pas glycosylée. Son rôle physiologique n'est pas encore totalement élucidé. Les animaux transgéniques dépourvus de cette protéine se montrent viables, mais présentant une réponse immunitaire amoindrie, les rendant plus sensibles vis-à-vis de certaines infections virales et parasitaires. La diminution de la réponse immunitaire chez ces animaux semble corrélée au fait que les cellules de ces animaux présentent très peu d'antigènes au niveau de leur complexe MHC I et peu de lymphocytes T fonctionnels.

Il semble, cependant, que la β2m soit un élément indispensable pour une présentation correcte des peptides antigéniques ("non soi") aux cellules effectrices (lymphocytes T CD8) [Townsend A., and Bodmer H. (1989) Ann. Rev. Immunol. 7:601-624; Hansson S.F. et al. (1989) Proteome Science 2: 1-11) et qu'elle soit également nécessaire à l'activation des lymphocytes T [Pereira P., et al., Blockade of transgenic gamma delta T cell development in beta 2-microglobulin deficient mice (1992) EMBO Journal 11:25-31].

II est ainsi établi depuis la fin des années 1980 que la β2m peut favorablement améliorer la réponse antigénique et être utilisée en tant qu'adjuvant de vaccin pour stimuler la réponse immunitaire liée aux lymphocytes T (CD8).

De nombreux documents indiquent que la β2m peut être ainsi incorporée dans des compositions vaccinales en combinaison avec des molécules chargées d'induire une réaction immune, tels que des antigènes spécifiques de virus ou de tumeurs.

La β2m aurait pour effet dans ces compositions d'augmenter la densité des complexes MHC I présentant des antigènes.

Dans de telles compositions vaccinales, la β2m peut être présente sous différentes formes, purifiée ou recombinante. Cependant, elle se trouve toujours en combinaison avec les antigènes destinés à induire la réponse immunitaire.

Des constructions génétiques sont également décrites dans lesquelles le gène codant la β2m est fusionné avec des séquences génétiques codant des peptides immunogènes dans le but d'exprimer *in-vivo* des protéines de fusion destinés à éliciter une réaction immunitaire spécifique [WO 99/64957].

Ces compositions vaccinales et autres vecteurs d'immunisation sont destinées à être utilisées en immunothérapie, plus particulièrement pour le traitement de cancers ou d'infections virales.

On retiendra que dans ces compositions vaccinales, la β2m est toujours utilisée en tant qu'adjuvant et non en tant que principe actif. Cela tient sans doute au fait, qu'à ce jour, aucun effet thérapeutique de la β2m n'a été constaté pouvant justifier de son emploi dans des compositions pharmaceutiques.

La β2m est décrite, au contraire, comme étant impliquée ou associée à diverses pathologies.

Ainsi, le syndrome d'immunodéficience dans la maladie du SIDA, qui peut se manifester de nombreuses années après l'infection par le **VIH,** est précédé d'une augmentation brutale du taux de β2m dans le sang.

Certaines publications [Wu C.H. et al. (2001) Oncogene 20 :7006-20] soulignent que l'augmentation du taux de β2m est corrélée et, peut-être, impliquée dans le développement de certains cancers, notamment le cancer des os et de la prostate [Gross M. et a/. (2007) Clin. Cancer Res., 13:1979-1986]. Pour d'autres cancers, des baisses du taux de β2m sérique sont observées, comme dans le cancer du colon [Kaklamanis L. et al. (1992) lnt. J. Cancer 57:379-385].

Le dosage de la β2m dans le sang (plus précisément le sérum sanguin), le liquide céphalorachidien ou la salive, est fréquemment utilisé dans le diagnostic de certaines maladies infectieuses ou parasitaires mais aussi, à titre principal, pour le diagnostic de certaines maladies du rein, du système lymphatique, des rhumatismes, des maladies inflammatoires, des maladies neurologiques, telles que la maladie d'Alzheimer et la démence fronto-temporale [Davidsson P. et al., Proteome analysis of cérébrospinal fluid proteins in Alzheimer patients (2002) Clinical Neuroscience and Pathology 13: 611-615 ; Hansson S.F. et al., Validation of a prefractionation method followed by two-dimensional electrophoresis-Applied to cerebrospinal fluid protein from frontotemporal dementia patients (2004) Proteome Science 2 :1-11].

Chez les personnes considérées comme en bonne santé, le taux moyen de β2m dans le sang reste relativement constant, inférieur ou égal à 2 mg/I, ce qui n'est pas le cas des maladies visées ci-dessus, où ce taux peut atteindre des valeurs aussi élevées que 4.0 mg/1.

Pour certaines pathologies, l'augmentation de la β2m sérique pourrait être causée par un "shedding" (relargage de protéines de la surface cellulaire) accru de la β2m des lymphocytes [Bellotti V., et al. (1999) Cell. Mol. Life Sci., 55-977-991].

La β2m plasmatique circulant dans le sang est normalement filtrée au niveau des reins par les glomérules, puis réabsorbée et catabolisée au niveau tubulaire.

Des études ont montré que la moitié de la β2m plasmatique (forme libre de la β2m), renouvelée chaque jour, provient des lymphocytes, et plus particulièrement du recyclage des complexes MHC. Ce renouvellement contribuerait ainsi à lui seul à une production importante de β2m sérique d'environ 150 mg/24 h pour une personne de taille moyenne. Cependant, le "turn over" en stabiliserait le taux sérique à 2 mg/I.

Chez les patients sous dialyse, chez lesquels la β2m n'est pas éliminée par les reins, l'accumulation de β2m dans les fluides corporels a des conséquences délétères. En particulier, elle induit des arthropathies et des neuropathies par formation de plaques amyloïdes au niveau de certains tissus conjonctifs (nerveux et articulaires) [Ohshi K., et al. Pathogenesis of beta2-microglobulin amyoidosis (2001) Pathol. lnt. 51 :1-10].

Dans l'ostéoarthrite (arthrose), la β2m est décrite pour avoir un effet inhibiteur sur la prolifération des chondrocytes, ayant pour conséquence d'accentuer la destruction des cartilages [WO 2004/020586].

Dans le cas de certaines maladies auto-immunes, telle que la sclérose en plaques (SEP), il est courant de suivre l'évolution du taux de β2m chez les patients, pour anticiper la survenue de crises inflammatoires [Bagnato, F., beta-2 microglobulin and neopterin as markers of disease activity in multiple sclerosis (2003) Neurol. Sci. 24 :51301-51304]. Le taux de β2m est alors préférentiellement mesuré dans le liquide céphalorachidien car le taux de β2m dans le sang est considéré comme trop fluctuant [Caudie C. et a/. Valeurs usuelles et utilité diagnostique de la β2-microglobuline dans le liquide céphalorachidien (2005) Ann. Biol. Clin. 63(6):631-637; Ryu O.H., et al. (2006) Rheumatology, 45:1077-1086].

L'implication de la β2m dans les maladies auto-immunes reste imprécise et mériterait d'être davantage étudiée.

Les maladies auto-immunes forment un large ensemble de maladies dont les symptômes peuvent être attribués à une hyperactivité du système immunitaire, avec présence ou non d'auto-anticorps, dirigés à l'encontre de substances ou de tissus qui sont normalement présents dans l'organisme.

II est certain que la réponse immunitaire contre le « soi », dans les maladies auto-immunes, résulte de l'activation des lymphocytes T via le système CMH et plusieurs mécanismes peuvent en être la cause.

Au niveau du système immunitaire :
- Par induction d'auto-anticorps à l'aide de cellules T par présentation d'antigènes reconnus par lesdits auto-anticorps. C'est le cas du lupus erythémateux disséminé (LED), de la thyroïdite de Hashimoto, de la sclérose en plaques (SEP), du diabète insulino-dépendant (type I) etc.

Au niveau cellulaire :
- Induction d'une réponse auto-immune par activation de cellules T spécifiques d'un antigène viral ;
- Altération au niveau de la reconnaissance CPA-MHC-I /TcR (récepteur aux cellules T) et au niveau de la cascade de signalisation(s) du lymphocyte T activé ;
- Mauvais assemblage dans la CPA des composantes du système MHC-I ;
- Défaut(s) dans le fonctionnement des cellules régulatrices.

Au niveau moléculaire :
- Mimétisme moléculaire ou tolérance ;
- MHC I comme auto-antigène;

Cependant, les causes exactes des nombreuses maladies auto-immunes ne sont toujours pas élucidées.

La plupart des maladies auto-immunes sont considérées comme résultant d'une conjonction d'une prédisposition génétique et d'un épisode infectieux au cours duquel l'organisme développe une immunité vis-à-vis de ses propres antigènes.

Les maladies auto-immunes les plus répandues sont la polyarthrite rhumatoïde, le lupus érythrodermique systémique, la sclérodermie, la fibromyalgie, la myosite, la spondylarthrite ankylosante, le diabète insulino-dépendant de type I, la maladie de Crohn et la sclérose en plaques (SEP).

Il existe de nombreuses autres affections, parmi les maladies dites « orphelines », suspectées d'être également des maladies auto-immunes. La sclérose latérale amyotrophique (SLA) fait partie de ces maladies, pour lesquelles aucun remède efficace n'est actuellement disponible.

II convient de distinguer deux types de maladies auto-immunes : les maladies auto-immunes spécifiques et les maladies auto-immunes non spécifiques.

Dans les maladies non spécifiques, différents organes sont touchés, causant des maladies systémiques tels que l'arthrite rhumatoïde, le lupus érythrodermique systémique, le syndrome de Sjögren et la sclérodermie.

Les maladies spécifiques se limitent plus particulièrement à certains organes. Les plus communes sont le diabète insulino-dépendant, les maladies de la thyroïde, la maladie d'Addison, quelques maladies des reins, des poumons, et surtout, la sclérose en plaques.

Les thérapies actuelles présentent une gamme d'approches allant de l'inflammatoire aux immunosuppresseurs en passant par les antimétabolites et les anticancéreux. A titre d'exemple sont utilisés : les anti-inflammatoires non-stéroïdiens (AINS), les glucocorticoïdes, les antimétabolites (méthotrexate, azathioprine), le cyclophosphamide, la sulfasalazine, les sels d'or, la cyclosporine A, le mycophénolate, le léflunomide.

Récemment, l'interféron β a été préconisé pour la SEP et des dérivés de chloroquine (utilisés contre la malaria) sont préconisés pour les traitements du lupus érythémateux et de la polyarthrite rhumatoïde.

Ces traitements, utilisés pour traiter d'autres maladies, ne sont pas très adaptés et présentent de nombreux effets secondaires gênants, en particulier, lorsqu'ils sont utilisés sur le long terme. Par ailleurs, s'ils parviennent à atténuer, en partie, les symptômes de ces maladies, ils ne permettent pas d'obtenir la rémission des malades.

L'inventeur désigné dans la présente demande, s'est plus particulièrement intéressé à la situation de deux patientes atteintes de maladies auto-immunes apparemment distinctes :
- Une première patiente (Pl) souffrant d'une maladie auto-immune non spécifique, c'est-à-dire n'affectant pas un organe précis, mais souffrant aussi de la thyroïdite d'Hashimoto et du syndrome de Sjôrgen primaire ; et
- Une seconde patiente (P2) souffrant de SEP.

Pour ces patientes, l'inventeur s'est attaché à établir le rapport des quantités CL (MHC-ABC)/ β2m provenant de lymphocytes, isolés à partir du sang des malades selon les méthodes conventionnelles indiquées plus loin.

De manière surprenante, ce rapport βCL/(32m s'est montré en forte augmentation chez ces deux patientes, comparé à celui de donneurs témoins, alors que le taux de β2m sérique ne dépassait pas la normale: environ 1,8 mg/I pour P1 et 1,5 mg/I pour P2.

Les résultats de ces mesures montrent un déséquilibre dans le rapport CL/β2m. Ils ont révélé une situation inattendue, selon laquelle les complexes MHC présents chez ces deux patientes seraient déficitaires en β2m par rapport au taux de CL, sans que cela n'augmente le taux de β2m libre dans le sang.

Ces résultats ont interpellé l'inventeur et l'ont amené à faire l'hypothèse que les deux maladies auto-immunes dont sont atteintes ces deux patientes, pourraient avoir pour origine commune un déficit de β2m au niveau des complexes MHC I. De manière plus générale, un déséquilibre CL/β2m au niveau des complexes MHC contribuerait à la manifestation des troubles rencontrés dans de nombreuses maladies auto-immunes.

Selon cette hypothèse développée plus loin, la réaction auto-immune dans le cadre des pathologies dont souffrent les deux patientes ne serait pas la conséquence d'une augmentation générale de la β2m, mais au contraire, aurait pour origine un déficit local de β2m au niveau des complexes MHC I membranaires susceptible d'altérer la présentation des antigènes aux cellules T(CD8).

On notera que cette hypothèse n'exclut en rien l'implication de la β2m dans l'activation des lymphocytes T et dans le processus inflammatoire, telle qu'elle a pu être décrite dans l'art antérieur.

Compte tenu de ces premières observations, l'inventeur a procédé à l'analyse des complexes MHC présents chez d'autres patientes atteintes de SPE et d'autres maladies auto-immunes comme la maladie de Crohn. Il a ainsi pu constater que le rapport CL/β2m provenant de lymphocytes de ces patients était supérieur à celui de patients témoins.

Sur la base de ces résultats, l'inventeur a mis au point des compositions pharmaceutiques dont la β2m est le principal principe actif.

Le but de ces compositions est de pallier un déficit en β2m des complexes MHC I chez les patients atteints de maladies auto-immunes.

**Figure 1** : Représentation schématique d'un complexe MHC de type dans un plan (A) et dans l'espace (**B**). La chaîne lourde (CL) est constituée de 3 domaines extracellulaires (α1, α2 et α3) et d'un domaine transmembranaire. La chaine légère β2m), qui est extracellulaire s'intercale entre la membrane et le rapprochement entre les domaines α1 et α3 de la chaine lourde. La figure B montre la position des peptides (antigènes) présentés par la chaîne lourde.

**Figure 2** : Photographie (x 630) de lymphocytes mis en contact avec des liposomes conformes à l'invention. Les liposomes (tâches claires) s'adsorbent sur la membrane des lymphocytes (HLA-ABC). Le noyau cellulaire est intègre (gris).

**Figure 3** : Photographie (x 630) de lymphocytes mis en contact avec des liposomes conformes à l'invention. La protéine (tâches plus sombres) contenue dans les liposomes est transportée dans les lymphocytes (HLA-ABC).

### DESCRIPTION DETAILLEE

La présente invention a donc pour objet une utilisation de la protéine β2m en tant que principe actif, notamment pour la préparation d'un médicament.

La protéine β2m est de préférence la forme humaine de la protéine, purifiée ou recombinante, dont la séquence polypeptidique ainsi que les déterminants génétiques sont décrits dans la base de donnée GENEBANK, sous le numéro d'accession CAG33347. Lorsque la protéine est produite de manière recombinante, il est recommandé pour la synthèse de la protéine d'utiliser des cellules hôte eucaryotes permettant une glycosylation de la protéine proche de celle de la protéine humaine.

Dans certains cas, la β2m peut être purifiée à partir de sera de donneurs sains, dans d'autres cas, elle peut être utilisée sous forme de fragments purifiés synthétisés chimiquement (synthèse peptidique).

Dans la mesure où ils sont tolérés par l'organisme, des variants fonctionnels de la protéine β2m peuvent également être utilisés.

Par variant fonctionnel, on désigne un polypeptide capable de s'associer aux complexes MHC présents à la surface des cellules, dont la séquence polypeptidique est au moins 70 %, de préférence au moins 80 %, plus préférentiellement au moins 90 %, et encore plus préférentiellement au moins 95 %, identique à la séquence polypeptidique de la de la protéine β2m humaine (la comparaison de séquence étant effectuée, par exemple, à l'aide du logiciel ClustalW). Un variant fonctionnel de la β2m peut également consister en un fragment de cette protéine présentant le même effet thérapeutique.

De tels variants fonctionnels peuvent présenter certains avantages au titre de l'efficacité du produit ou de sa formulation par rapport à la protéine humaine purifiée. lls peuvent également résulter de l'expression de séquences nucléotidiques clonées dans un vecteur d'expression ou de thérapie génique.

De nombreuses publications décrivent, par exemple, la présence d'isoformes de β2m chez les rongeurs [Goding J.W. et Walker I.D. Allelic forms of β2-microglobulin in mouse (1980) Proc. Natl. Acad. Sci. USA 77: 7395-7399] et chez l'humain [Davidsson P. et al., Proteome analysis of cérébrospinal fluid proteins in Alzheimer patients (2002) Clinical Neuroscience and Pathology 13: 611-615 ; Hansson S.F. et al., Validation of a prefractionation method followed by two-dimensional electrophoresis-Applied to cerebrospinal fluid protein from frontotemporal dementia patients (2004) Proteome Science 2 :1-11]. Ces isoformes, qui se distinguent plus particulièrement par un pH isoélectrique (pHi) différent, sont considérés dans leur utilisation selon l'invention, comme des équivalents de la β2m.

Les compositions pharmaceutiques comprenant de la β2m ou un des variants fonctionnels de la β2m, en tant que principe actif, sont objet de la présente invention. La β2m ou son variant fonctionnel forment, de préférence, le seul principe actif desdites compositions.

Au sens de la présente invention, un principe actif est une substance qui entre dans la composition d'un médicament et qui est responsable des propriétés pharmacodynamiques ou thérapeutiques de celui-ci. Un adjuvant n'est pas considéré au sens de la présente invention comme un principe actif.

Plus préférentiellement, l'invention a pour objet une composition pharmaceutique consistant en de la β2m ou un variant fonctionnel de la β2m contenu dans un support pharmaceutiquement acceptable, ledit support pharmaceutiquement acceptable étant préférentiellement un liposome.

En outre, l'invention a pour objet une méthode de thérapie génique comprenant une étape d'expression *in-vivo* ou *ex-vivo* de la β2m ou d'un variant fonctionnel de celle-ci, en tant que principe actif. Elle a corrélativement pour autre objet, un vecteur de thérapie génique comprenant une séquence génétique permettant l'expression de la β2m ou dudit variant fonctionnel de celle-ci. Différents types de vecteurs viraux ou non viraux sont décrits dans la littérature pouvant être adaptés à cet effet [Urnov et al. (2005) Highly efficient endogenous human gene correction using designed zinc-finger nucleases, Nature, 435:577-579]. De préférence, le vecteur de thérapie génique selon l'invention permet l'expression dans le corps humain de la β2m (ou de son variant fonctionnel) dans le corps humain à l'exception de tout autre principe actif, et préférentiellement de tout autre polypeptide.

Selon un aspect préféré de l'invention, la β2m est administrée seule dans un support pharmaceutiquement acceptable, tel qu'un liposome ou une solution physiologique, dans le respect des recommandations et exigences réglementaires.

Selon l'invention, la β2m est plus particulièrement utilisée pour sa capacité à restaurer un rapport normal CL/β2m au sein des complexes MHC-l chez un patient.

Le rapport CL/β2m est traité de préférence au niveau des cellules lymphocytaires. Le rapport CL/β2m correspond au rapport molaire des sous-unités CL et β2m au niveau des complexes MHC I purifiés.

De préférence, ce rapport est ramené à un niveau comparable à celui d'un patient non atteint par une maladie. Plus préférentiellement la β2m est utilisée dans le but de diminuer le rapport CL/β2m chez un patient pour atteindre un rapport molaire de 1.

La présente invention a plus particulièrement pour but de prévenir un déficit de β2m de se produire au niveau des complexes MHC chez des patients atteints de maladie auto-immunes.

L'inventeur a pu déterminer qu'un tel déficit peut occasionner un rapport CL/β2m supérieur à 2 chez les patients atteints de maladies auto-immunes. L'invention a donc pour but de ramener ledit rapport CL/β2m à une valeur préférentiellement inférieure à 2, plus préférentiellement inférieure à 1.5, et encore plus préférentiellement inférieure à 1,2.

L'invention peut bien entendu s'appliquer à toute maladie liée à un déséquilibre du rapport CL/β2m dans les complexes MHC l, autre que les maladies auto-immune.

Au sens de la présente invention, les pathologies liées aux transplantations d'organes ou aux rejets de greffe, ne sont pas considérées comme des maladies auto-immunes, ni comme des maladies causées par un défaut de reconnaissance du « soi » par le système immunitaire. En effet, le rejet de greffe est considéré ici comme résultant d'une reconnaissance du « non-soi » par le système immunitaire, et non d'un défaut de reconnaissance du « soi ».

Les analyses effectuées par l'inventeur indiquent que les maladies pour lesquelles le rapport CL/β2m est davantage susceptible d'occasionner des troubles, sont la polyarthrite rhumatoïde, le lupus érythrodermique systémique, le syndrome de Sjögren, la sclérodermie, la fibromyalgie, la myosite, la spondylarthrite ankylosante, le diabète insulino-dépendant de type I, la thyroïdite d'Hashimoto, la maladie d'Addison, la maladie de Crohn et la sclérose latérale amyotrophique (SLA).

Bien que la question soit encore débattue au sein de la communauté scientifique, il est considéré aux fins de la présente invention, et au vu des résultats obtenus par les inventeurs, que la sclérose latérale amyotrophique (SLA) est une maladie de type auto-immune.

L'utilisation de la β2m selon l'invention est donc plus particulièrement destinée au traitement des maladies auto-immunes.

L'invention vise plus particulièrement la mise au point d'un médicament pour augmenter le taux de β2m sanguin à une concentration comprise 2,5 et 6 mg/1, de préférence entre 3 et 5 mg/I, plus préférentiellement entre 3,5 et 4,5 mg/I.

Comme décrit ci-après, dans la partie expérimentale de la présente demande, le médicament selon l'invention, peut consister en une préparation liposomale comprenant de la β2m ou un variant fonctionnel de celle-ci. Les liposomes peuvent être fabriqués à l'aide de différentes techniques connues de l'homme du métier. Différents lipides constituant les liposomes peuvent être utilisés [Medical Application of Liposomes (1986) edited by Kunio Yagi, Japan Scientific Societies Press , Tokyo, Karger].

Un médicament préféré de l'invention consiste à cet égard en un liposome chargé en β2m. De préférence, la β2m ou un variant fonctionnel de cette protéine constituent les seuls principes actifs contenus dans ladite préparation liposomale.

Selon un autre aspect de l'invention, c'est un vecteur de thérapie génique codant pour la β2m ou son variant fonctionnel, tel que décrit précédemment, qui est formulé sous forme liposomale.

Il est avantageux d'utiliser un liposome selon l'invention en tant que médicament car il permet, d'une part, de protéger la β2m d'éventuelles attaques protéolytiques, et d'autre part, de délivrer la β2m de manière ciblée au niveau des complexes MHC I, notamment par fusion du liposome avec les phospholipides, qui constituent les membranes cellulaires.

Selon un aspect de l'invention, un patient peut être traité par transfusion ou perfusion de lymphocytes ayant été préalablement mis en contact avec de la β2m. Cette mise en contact peut s'effectuer par une incubation *"ex vivo"* de lymphocytes extraits d'un échantillon de sang prélevé auparavant chez le même patient ou bien chez un autre patient.

Selon un aspect préféré de l'invention, le médicament comprenant la β2m est préparé sous une forme saline. Un procédé de préparation préféré du médicament consiste à incuber la β2m sous forme saline, *in-vitro,* au contact du sérum du patient auquel le médicament est destiné.

Les compositions pharmaceutiques selon l'invention décrites plus haut peuvent prendre toute forme appropriée pour leur administration orale, par injection, perfusion ou inhalation, connue de l'homme du métier.

Un autre aspect de l'invention concerne le diagnostic des maladies auto-immunes, plus particulièrement le diagnostic des maladies précédemment citées, par détermination *in-vivo* ou *in-vitro* du rapport CL/β2m des complexes MHC I.

Le procédé de diagnostic selon l'invention comprend, de préférence, une ou plusieurs des étapes suivantes consistant à :
i) prélever des cellules chez un patient chez lequel une maladie auto-immune est à dépister, de préférence des lymphocytes ;
ii) extraire les complexes MHC 1 de ces cellules, et si nécessaire;
iii) déterminer les quantités respectives de CL et de β2m contenues dans lesdits complexes ;
iv) établir le rapport molaire CL/β2m.
v) comparer le rapport CL/β2m obtenu avec les résultats préalablement obtenus chez d'autres patients.

De préférence, le procédé de diagnostic selon l'invention comprend une étape de comparaison du rapport CL/β2m avec celui d'un témoin, ou bien dans le cadre du suivi d'un patient, avec d'autres rapports préalablement déterminés.

Les quantités respectives des protéines de CL et β2m peuvent être déterminées de manière standard selon les méthodes connues de l'homme du métier, par exemple, par immuno-détection quantitative (ex : ELISA, lmmunodot, "Western Blot", biopuces à auto-antigènes etc.). L'extraction des complexes MHC l se pratique suivant les protocoles connus d'extraction des protéines membranaires.

Le procédé de diagnostic selon l'invention peut être mis en oeuvre dans le cadre d'un suivi thérapeutique des patients atteints de différentes maladies auto-immunes.

Les exemples ci-après ont pour but de compléter la description de l'invention sans en limiter la portée.

### EXEMPLES

### I - Analyse des composantes des systèmes HLA-I membranaires des lymphocytes chez des patients souffrant des maladies auto-immunes.

Sans être lié par la théorie, l'inventeur a émis l'hypothèse de travail qu'une augmentation du rapport CL/β2m peut avoir pour conséquence des réactions de type auto-immun.

En effet, un défaut d'assemblage ou une diminution de l'exposition du système MHC I vers le compartiment extracellulaire parait entraîner des réactions inflammatoires. Il pourrait donc en résulter que :
- Le système immunitaire ne reconnaît pas parfaitement le « non soi ». Une « sur-exposition » des composantes du système MHC pourrait alors entraîner une réaction auto-immune. La probabilité pour que cet état induise une reconnaissance du « soi » par le TcR (récepteur des cellules T) serait alors augmentée ; les composantes en excès amplifierait, de ce fait, les arcs MHC/TcR et TcR/cascades de la messagerie cellulaire/réponse(s) immunitaire(s).
- Au niveau des MHC l, un excès de CL, une diminution de β2m, ou les deux à la fois, pourraient entraîner un phénomène de « sur-exposition » du « soi » au TcR. Notons que la p2m protège des régions spécifiques des CL et façonne le « non soi ». Une altération dans ce « façonnage » est une porte ouverte à la présentation du « non soi ».

Pour vérifier cette hypothèse, une analyse a été effectuée pour déterminer les quantités molaires de CL et de β2m dans les complexes MHC l extraits du sang de deux patientes.
- la première patiente (Pl) souffre de la thyroïdite d'Hashimoto et d'un syndrome de Sjörgen primaire. Cependant, tout porte à croire que la malade souffre d'une maladie auto-immune non spécifique.
- la deuxième patiente (P2) souffre de la SEP.

Les lymphocytes ont été isolés à partir du sang des donneurs sains et des patients selon la méthode de Lightbody J. *[*Manual of Clinical lmmunology, Rose NR., Friedman H. Editors American Society for Microbiology Washington (DC), 1976, pages 851-857] modifiée par Hofman F.M. et al. [Ann. J. Clin. Pathol. (1982) 77:710-716]. Les complexes MHC-1 sont détectés sur les lymphocytes entiers ou sur les membranes plasmiques préparées selon la méthode de Warley A. et al. [Biochim. Biophys-Acta (1973), 323:55-66] avec quelques modifications. Dans certains cas, l'analyse a porté sur des "radeaux" ("rafts") selon Jennifer L. et al. [J. Lip. Res. (2005) 46:1061-1067]. La détection des composantes protéiques des MHC-1 a été effectuée par électrophorèse (système SDS-PAGE), selon Laemmli U.K. [Nature (1970) 227:680-685] puis par électro-transfert sur des membranes en PVDF et immuno-empreinte selon la méthode de Towbin H. et al. [Proc. Natl. Acad. Sci. USA (1979) 76:4350-4354]. Les révélations ont été réalisées par des anticorps secondaires couplés à la phosphatase alcaline à l'aide d'un mélange NBT-DCIP.

Il a été vérifié que l'excès des chaînes lourdes est bien membranaire (isolement des membranes plasmiques et/ou de microdomaines membranaires de type "raft" et démonstration du phénomène CL/β2m>1).

Les résultats obtenus montrent clairement un rapport CL/β2m (MHC I) nettement supérieur à 1 chez ces deux patientes (le rapport CL/β2m est d'environ 2,2 pour P1 et 4,0 pour P2).

D'autres cas de SEP, au nombre de quatre, et deux cas de maladie de Crohn montrent un même résultat en ce qui concerne le rapport CL/β2m. Ces observations ont incités le demandeur à élaborer une approche expérimentale permettant de restaurer l'équilibre CL/β2m (MHC-I).

### II - Préparation de liposomes chargés en β2m :

### Evaluation de la quantité de β2m à délivrer aux patients

Pour amener en excès la β2m à la surface des globules blancs, il convient d'augmenter sa concentration dans le sang dans les limites du "raisonnable" afin de ne pas enclencher des voies de signalisation sur des cellules possédant un potentiel de multiplication.

Vu la facilité avec laquelle la β2m se décroche des membranes et circule dans le système sanguin et rénal, il convient d'amener le taux de β2m sanguin entre 2.5 et 3.5 mg/I (le taux normal oscille autour de 2.0 mg/I de sang). Cette augmentation, en soi, peut favoriser l'adsorption de la β2m à la surface des cellules.

Notons que les complexes majeurs d'histocompatibilité de type I sont composés mole/mole de chaînes lourdes (PM= 45kDa) et de β2m (PM = 12kDa). Un complexe (PM ≈ 57kDa) est donc composé, en poids, de 74% de chaîne lourde et 21 % de chaîne légère.

La teneur moyenne en protéines d'un lymphocyte est de 650 x 10-¹² g et la teneur protéique de sa membrane plasmique ne représente que 1 % de la teneur totale, soit 6.5 x 10-¹² g. Si on considère que les MHCI ne représentent, au maximum, que 1% de la teneur totale en protéines membranaires d'un lymphocyte quiescent, la teneur en β2m est donc de 1.4 x 10⁻¹⁴ g environ par lymphocyte.

En prenant des paramètres physiologiques moyens, 2 x 10⁶ lymphocytes/ml de sang et 5 litres de sang par individu, la fourchette en "poids" du MHCI total/individu (concernant les lymphocytes) serait de 1.4 x 10-⁶ g à 1.8 x 10-⁶ g de β2m lymphocytaire. Ces chiffres sont vraiment maximaux car nos premières estimations montrent plutôt des valeurs de 500 x 10-⁹ g en moyenne.

La quantité moyenne dans le sang étant de 10 mg de β2m par individu, soit une quantité très largement supérieure celle pré sente à la surface des lymphocytes, il semble qu'il existe déjà, dans les conditions normales, un rapport entre β2m membranaire et β2m sérique largement inferieur à 1. Il n'est donc pas déraisonnable d'augmenter le taux de β2m dans la circulation sanguine (augmentation de sa pression partielle oncotique) pour combler le déficit membranaire en β2m.

L'administration de la β2m peut s'opérer de deux façons:
(1) administration de liposomes chargés en β2m. Ce type de vecteur est toujours en usage pour l'administration de peptides, anticorps, matériel génétique etc. L'utilisation de liposomes (membranes "artificielles" ou synthétiques) favorise le contact entre la surface cellulaire et le principe actif;
(2) Incubation du principe actif sous forme saline avec le sérum des malades avant administration. Le but de cette incubation est que les lipoprotéines du sérum agissent comme vecteur à l'instar des liposomes.

Le taux d'incorporation de β2m dans les lymphocytes suite à l'administration par la méthode 1 ou 2 peut être comparé à une administration contrôle; dans ce dernier cas, la perfusion saline β2m est administrée à raison de 0, 10mg/ml (volume total 150 ml), ce qui amènerait 3 mg de β2m par litre de sang.

### Formulation des liposomes

Pour la préparation des liposomes (pour 1 ml): après évaporation à sec sous azote de chlorure de méthylène (CH₂C1₂), un film contenant de la phosphatidylcholine, avec ajout ou non de cholestérol, avec ajout ou non de sphingomyéline ou avec ajout de cholestérol et de sphingomyéline. Pour les trois composés (phosphatidylcholine, cholestérol et sphingomyéline) les proportions sont respectivement de 10M, 2M et 1M - soit pour 1 ml de solution finale 7,60mg, 0,76mg et 0,76mg. A ce film est ajouté 1 ml d'une solution saline (PBS 10mM pH 7,4; HANKS, Tris/Glycine ou DMEM) contenant 2mg de β2m. La molarité reste la même pour chacun des composés si des liposomes faits de phosphatidylcholine (10M), de phosphatidylcholine (10M) et de cholestérol (2M), de phosphatidylcholine (10M) et de sphingomyéline (1M) sont fabriqués. Mais d'autres molarités concernant les composantes lipidiques peuvent être utilisées. Les quantités de protéines peuvent être autres et le pH peut être supérieur à 7.4 selon les cas. La dispersion du film lipidique est réalisée par agitation à 37°C pendant 3h. La solution est ensuite passée 4 fois à une pression de 450 Bars pour obtenir des SUV (small unilamellar vesicules). Au laboratoire, nous utilisons pour ce faire le système "Liposofast" (Sodexim S.A. 51140 Muizon France).

Dans des expériences «pré-pilotes», et ceci pour montrer l'incorporation de la β2m dans des liposomes, l'adsorption des liposomes sur la surface cellulaire et le transfert de la protéine du liposome vers l'intérieur de la cellule, nous avons procédé à la fabrication de liposomes fluorescents. En fonction des expériences, des liposomes ont été préparés, qui émettent une fluorescence à 520 nm ou 572 nm. Pour ce faire, 0.5 M de NBD-PC (1-oleyl-2-(-6-(((7-nitro-2-1,3-benzoxadiazol-4-yl)amino)hexanoyl)-sn-glycero-3-phosphocholine)(excitation à 490 nm et émission à 520 nm) ou 0.5 M de Liss Rhod PE (1,2-dioleyl-sn-glycero-3-phosphatidyletholamine-N-(lissamine rhodamine B sufonyl)(sel d'ammonium)(excitation à 541 nm et émission à 572 nm) ont été ajoutés au mélange lipidique avant évaporation et obtention du film lipidique (voir ci-dessus).

A l'échelle pré-pilote, les liposomes ont été fabriqués par la méthode d'extrusion. Par cette technique, nous avons obtenu aussi des SUV bien calibrés et stables.

Brièvement, après agitation à 37°C pendant 3h, la suspension micellaire est dialysée contre une solution saline contenant de la β2m ainsi que 4µM (0,8mg/ml) de n-hexyl- βD-gtucopyranoside pendant 12h à 4°C dans un appareil à microdialyse. Les membranes de dialyse ont une limite d'exclusion de 3.5 kDa et le n-hexyl-βD-glucopyranoside (détergent) est dilué d'au moins 1 pp millions dans les solutions finales.

Les liposomes obtenus ont une taille d'environ 200 nm de diamètre. Ils sont stables pendant 3 mois, au moins, à température ambiante et contiennent, au moins 0.1 mg de β2m/ml de solution.

Sous réserve de l'accord des autorités compétentes, il est prévu d'administrer les liposomes obtenus sous-forme de perfusion, à une posologie de 150ml de solution liposomale (en moyenne : 15mg de β2m) par adulte et par jour. Pour des solutions liposomales concentrées (1 mg de principe actif par ml), elles peuvent être diluées facilement dans une solution saline sans porter atteinte à la structure et la stabilité des liposomes. L'administration de la β2m, consisterait idéalement à introduire la solution liposomale au niveau du canal thoracique, site de circulation des globules blancs (voie lymphatique).

### Application de liposomes tests sur des lymphocytes maintenus en culture

L'incorporation de la protéine dans les liposomes et l'application des liposomes fabriqués selon le protocole décrit ci-dessus à partir de phosphatidylcholine avec de la Liss Rhod PE a été testé *ex-vivo.*

La protéine utilisée pour ce test (albumine) a été rendue fluorescente par un marquage à la fluorescamine dont la fluorescence est comparable à celle du DAPI (Di Amino Phenyl Indol ; excitation 372 nm et émission à 456 nm). A cet effet, de l'albumine cristallisée de sérum de boeuf a été rendue fluorescente à l'aide du couplage par covalence de la fluorescamine sur l'extrémité N-terminale de la protéine, selon le procédé décrit par Hames B.D. et al. [Gel Electrophresis of Proteins, a practical approach, Hames BD. and Rickwood D. eds. The practical Approach Series, 2nd Edition. IRL Press, Oxford, New York, Tokyo. p.67] excepté que le marquage s'est fait dans un tampon Tris-Hcl (25 mM) / Glycine (192 mM) (pH 8.3) ne contenant pas de détergent (SOS). Les liposomes formés, comme décrit précédemment, contiennent 2 mg d'albumine fluorescente par ml de solution liposomale.

Ensuite, 0.2 ml (Hank's/0.5 mM EDTA, pH 7.4), contenant 250 000 lymphocytes, ont été incubés avec 0.2 ml de liposomes (Tampon 25 mM Tris-Hcl/ 192 mM Glycine, pH 8.3) pendant 1 heure à 37°C dans une atmosphère humide saturée en C02 (5 %).

Finalement, des liposomes contrôles (200 µl), des lymphocytes contrôles (200 µl) et des lymphocytes traités par des liposomes chargés en protéine (200 µl) ont été sédimentés sur des lamelles traitées et recouvertes par de la polylysine, et de la laminine, selon le procédé décrit par Rakotoraivelo C et al. [Receptors to steroid hormones and aromatase are expressed by cultured motoneurons but not by glial cells derived from rat embryo spinal cord (2004) Neuroendocrinology 80 :284-297].

Les préparations ont été observées directement au microscope à épifluorescence (Axiovert ; Zeiss, Allemagne) ou fixées avec une solution à 4% (plv) de paraformaldhéhyde dans de l'eau pendant 30 minutes, les lamelles étant rincées 3 fois avec du PBS (160 mM, pH 7.2). Les préparations ont été perméabilisées, de manière douce, avec 0.1 % de Triton X-100 (v/v), préparé dans du PBS, pendant 5 min.

Les cellules ont été marquées par des anticorps primaires anti HLA-ABC. Dans certains cas, les noyaux cellulaires ont été marqués au Hoechst (fluorescence 25 bleue, OAPI). Les anticorps primaires, produits chez le lapin, sont les mêmes que ceux utilisés pour le « Western Blot ». Les anticorps primaires sont dilués au 1/50 et les secondaires couplés au FITC (fluorescence verte) et produits chez la chèvre sont dilués au 1/160. Les incubations des anticorps ont été effectuées dans du PBS contenant 2% d'Albumine de sérum de boeuf. Pour l'objectif x63, les lamelles ont été montées avec du Fluorsave (Calbiochem, USA).

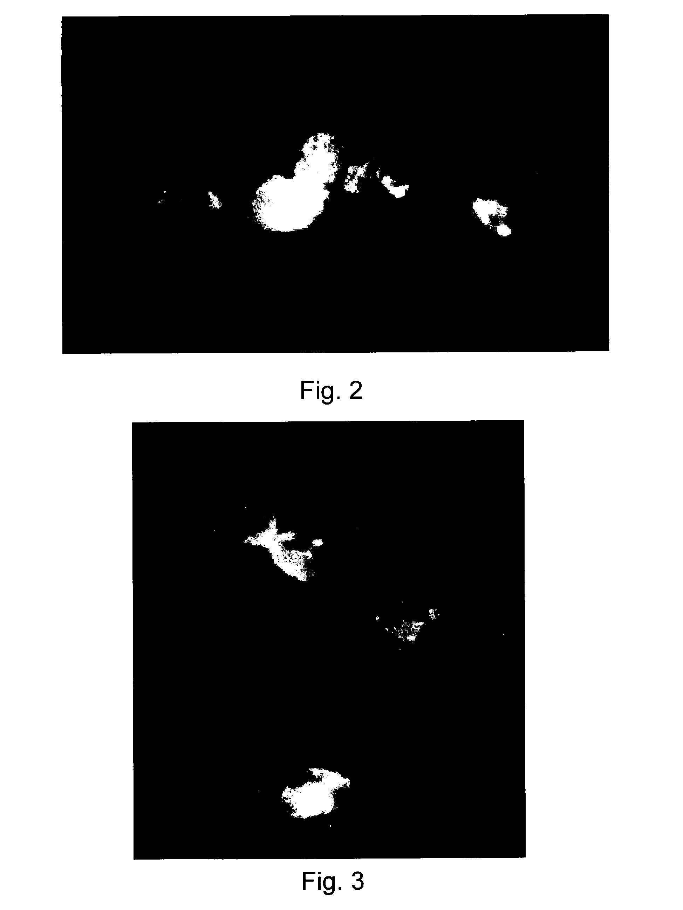

Les observations, illustrées par les photographies des figures 2 et 3, montrent que les liposomes sont adsorbés à la surface des lymphocytes et que la protéine marquée, contenue dans les liposomes, est déposée à la surface de la membrane desdits lymphocytes.

## Revendications

1. β2m ou variant fonctionnel de cette protéine pour leur utilisation en tant que médicament.

2. Médicament, **caractérisé en ce qu'**il comprend de la β2m ou un variant fonctionnel de cette protéine, en tant que principe actif.

3. Médicament selon la revendication 2, dans lequel le principe actif est la protéine β2m humaine.

4. Médicament selon la revendication 2, dans lequel le principe actif est un variant fonctionnel de la β2m présentant au moins 70 %, de préférence 80 %, et plus préférentiellement 90% d'identité avec la protéine β2m humaine.

5. Médicament selon l'une des revendications 1 à 4, pour le traitement d'une maladie auto-immune.

6. Médicament selon l'une des revendications 1 à 5, pour le traitement de la sclérose latérale amyotrophique.

7. Utilisation de la β2m ou d'un variant fonctionnel de cette protéine pour la préparation d'un médicament destiné au traitement d'une maladie auto-immune.

8. Utilisation selon la revendication 7, dans laquelle la maladie auto-immune est la polyarthrite rhumatoïde, le lupus érythrodermique systémique, le syndrome de Sjögren, la sclérodermie, la fibromyalgie, la myosite, la spondylarthrite ankylosante, le diabète insulino-dépendant de type I, la thyroïdite d'Hashimoto, la maladie d'Addison, la maladie de Crohn, la sclérose en plaques ou la sclérose latérale amyotrophique.

9. Utilisation selon la revendication 7, pour la préparation d'un médicament destiné au traitement de la sclérose latérale amyotrophique.

10. Utilisation selon la revendication 7, pour la préparation d'un médicament destiné au traitement de la sclérose en plaques.

11. Utilisation selon la revendication 7, pour la préparation d'un médicament destiné au traitement de la maladie de Crohn.

12. Utilisation selon la revendication 7, pour la préparation d'un médicament destiné au traitement de la polyarthrite rhumatoïde.

13. Utilisation selon la revendication 7, pour la préparation d'un médicament destiné au traitement du diabète de type I.

14. Utilisation selon l'une des revendications 7 à 13, dans laquelle le médicament est destiné à augmenter le taux de β2m sanguin à une concentration comprise entre 2,5 et 6 mg/I, de préférence entre 3 et 5 mg/1, plus préférentiellement entre 3,5 et 4,5 mg/I.

15. Utilisation selon l'une des revendications précédentes, dans laquelle le médicament consiste en un liposome chargé en β2m.

16. Utilisation selon l'une des revendications précédentes, dans laquelle la β2m est le seul principe actif contenu dans le médicament.

17. Utilisation selon l'une des revendications précédentes, **caractérisée en ce que** le médicament comprenant la β2m est préparé sous forme saline, et incubé préalablement ex-vivo au contact du sang, du sérum, ou des lymphocytes du patient à traiter.

18. Liposome chargé avec de la β2m pour le traitement des maladies auto-immunes.

19. Liposome selon la revendication 18, **caractérisé en ce qu'**il est destiné au traitement de l'une des maladies auto-immunes suivantes : la polyarthrite rhumatoïde, le lupus érythrodermique systémique, le syndrome de Sjögren, la sclérodermie, la fibromyalgie, la myosite, la spondylarthrite ankylosante, le diabète insulino-dépendant de type I, la thyroïdite d'Hashimoto, la maladie d'Addison, la maladie de Crohn, la sclérose en plaques ou la sclérose latérale amyotrophique.

20. Composition pharmaceutique comprenant un liposome selon l'une des revendications 18 ou 19.

21. Composition pharmaceutique comprenant de la β2m ou un variant fonctionnel de cette protéine en tant que principe actif pour le traitement d'une maladie auto-immune.

22. Procédé de diagnostic d'une maladie auto-immune, **caractérisé en ce qu'**il comprend une étape consistant à déterminer le rapport CL/β2m des complexes MHC de type I extraits d'un patient.

23. Procédé selon la revendication 19, **caractérisé en ce qu'**il comprend les étapes consistant à :
i) prélever des cellules chez un patient chez lequel une maladie auto-immune est à dépister, de préférence des lymphocytes ;
ii) extraire les complexes MHC I de ces cellules ;
iii) déterminer les quantités respectives de CL et de β2m contenues dans lesdits complexes MHC I;
iv) établir le rapport molaire CL/β2m desdits complexes MHC I ; ;
v) comparer le rapport CL/β2m obtenu avec celui d'un échantillon témoin.

## Revendications modifiées

### Revendications modifiées conformément à la règle 137(2) CBE.

**1.** Produit pharmaceutique **caractérisé en ce qu'**il comprend de la β2m ou un variant fonctionnel de cette protéine, en tant que principe actif.

**2.** Produit pharmaceutique **caractérisé en ce qu'**il comprend de la β2m ou un variant fonctionnel de cette protéine, en tant qu'unique principe actif.

**3.** Produit pharmaceutique **caractérisé en ce qu'**il consiste en la β2m ou un variant fonctionnel de cette protéine dans un support pharmaceutiquement acceptable.

**4.** Produit pharmaceutique selon l'une des revendications 1 à 3, dans lequel le principe actif est la protéine β2m humaine.

**5.** Produit pharmaceutique selon l'une des revendications 1 à 4, dans lequel le principe actif est un variant fonctionnel de la β2m présentant au moins 70 %, de préférence 80 %, et plus préférentiellement 90% d'identité avec la protéine β2m humaine.

**6.** Produit pharmaceutique selon l'une des revendications 1 à 5, dans son utilisation pour le traitement d'une maladie auto-immune.

**7.** Produit pharmaceutique selon la revendication 6, **caractérisé en ce** la maladie auto-immune traitée est la polyarthrite rhumatoïde, le lupus érythrodermique systémique, le syndrome de Sjögren, la sclérodermie, la fibromyalgie, la myosite, la spondylarthrite ankylosante, le diabète insulino-dépendant de type I, la thyroïdite d'Hashimoto, la maladie d'Addison, la maladie de Crohn, la sclérose en plaques ou la sclérose latérale amyotrophique.

**8.** Produit pharmaceutique selon l'une des revendications 1 à 7, dans son utilisation pour le traitement de la sclérose latérale amyotrophique.

**9.** Produit pharmaceutique selon l'une des revendications 1 à 7, dans son utilisation pour le traitement de la sclérose en plaques.

**10.** Produit pharmaceutique selon l'une des revendications 1 à 7 dans son utilisation pour le traitement de la maladie de Crohn.

**11.** Produit pharmaceutique selon l'une des revendications 1 à 7 dans son utilisation, pour le traitement de la polyarthrite rhumatoïde.

**12.** Produit pharmaceutique selon l'une des revendications 1 à 77, dans son utilisation pour le traitement du diabète de type I.

**13.** Produit pharmaceutique selon l'une des revendications 1 à 12, dans son utilisation pour augmenter le taux de β2m sanguin à une concentration comprise entre 2,5 et 6 mg/l, de préférence entre 3 et 5 mg/l, plus préférentiellement entre 3,5 et 4,5 mg/l.

**14.** Produit pharmaceutique selon l'une quelconque des revendications 1 à 13, dans son utilisation pour abaisser le rapport molaire CL/β2m des complexes MHCl chez un patient.

**15.** Produit pharmaceutique selon l'une quelconque des revendications 1 à 13, pour le traitement de patient souffrant d'un déficit de β2m au niveau des complexes MHCl.

**16.** Produit pharmaceutique selon l'une quelconque des revendications 1 à 13, **caractérisé en ce qu'**il consiste en un liposome chargé de β2m ou d'un variant fonctionnel de cette protéine.

**17.** Produit pharmaceutique selon l'une des revendications 1 à 14, **caractérisé en ce qu'**il est préparé sous forme saline et incubé préalablement ex-vivo au contact du sang, du sérum, ou des lymphocytes du patient à traiter.

**18.** Composition comprenant un produit pharmaceutique selon l'une des revendications 1 à 15.

**19.** Procédé de diagnostic d'une maladie auto-immune, **caractérisé en ce qu'**il comprend une étape consistant à déterminer le rapport CL/β2m des complexes MHC de type I extraits d'un patient.

**20.** Procédé selon la revendication 19, **caractérisé en ce qu'**il comprend les étapes consistant à :
i) prélever des cellules chez un patient chez lequel une maladie auto-immune est à dépister, de préférence des lymphocytes ;
ii) extraire les complexes MHC I de ces cellules ;
iii) déterminer les quantités respectives de CL et de β2m contenues dans lesdits complexes MHC I;
iv) établir le rapport molaire CL/β2m desdits complexes MHC I ;
v) comparer le rapport CL/β2m obtenu avec celui d'un échantillon témoin.
